Europäisches Patentamt

European Patent Office

Office européen des brevets

(11) Veröffentlichungsnummer: **0 014 823**
**B1**

(12) # EUROPÄISCHE PATENTSCHRIFT

(45) Veröffentlichungstag der Patentschrift:
**09.12.81**

(51) Int. Cl.³: **A 61 B 17/18**

(21) Anmeldenummer: **80100062.1**

(22) Anmeldetag: **07.01.80**

(54) **Implantat zur Fixation von Rippenbrüchen.**

(30) Priorität: **06.02.79 DE 7903184 U**

(43) Veröffentlichungstag der Anmeldung:
**03.09.80 Patentblatt 80/18**

(45) Bekanntmachung des Hinweises auf die Patenterteilung:
**09.12.81 Patentblatt 81/49**

(84) Benannte Vertragsstaaten:
**AT BE CH DE FR GB IT LU NL SE**

(56) Entgegenhaltungen:
**CH-A-611 147**
**FR-A-590 290**
**US-A-2 583 896**
**US-A-2 952 254**
**US-A-4 146 022**

(73) Patentinhaber: **Howmedica International, Inc.**
**Zweigniederlassung Kiel,**
**Professor-Küntscher-Strasse 1-5, D-2301 Schönkirchen**
**üb. Kiel (DE)**

(72) Erfinder: **Vecsei, V., Dr., Marokkanergasse 3/i/39 a,**
**A-1030 Wien (AT)**
Erfinder: **Richter, Karl M., Dipl.-Ing., Haferkamp 14,**
**D-2304 Wendtorf (DE)**
Erfinder: **Harder, Hans Erich, Mecklenburger Strasse 32,**
**D-2301 Probsteierhagen (DE)**
Erfinder: **Behrens, Klaus, Am Sportplatz 8,**
**D-2351 Rickling (DE)**

(74) Vertreter: **Hauck, Hans, Dipl.-Ing. et al, Patentanwälte**
**Dipl.-Ing.H.Hauck, Dipl.-Phys.W.Schmitz,**
**Dipl.-Ing.E.Graalfs, Dipl.-Ing.W.Wehnert,**
**Dipl.-Phys.W.Carstens Dr.-Ing.W.Döring Neuer Wall 41,**
**D-2000 Hamburg 36 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Implantat zur Fixation von Rippenbrüchen

Die Neuerung bezieht sich auf ein Implantat zur Fixation von Rippenbrüchen und damit zur Beseitigung der paradoxen Beweglichkeit des Brustkorbes.

Thoraxwandfrakturen mit paradoxer Atmung stellen aufgrund der Störung der ventilatorischen Lungenleistung einen lebensbedrohlichen Zustand dar. Folgende pathophysiologische Veränderungen sind dabei festzustellen:
Die Totraumbelüftung, die Atemfrequenz, der Atemwegwiderstand, die Atemarbeit, das Shuntvolumen nehmen zu und die alveoläre Kohlendioxidspannung und die Kohlendioxidausscheidung pro Minute nehmen ebenso ab wie das Herzminutenvolumen sowie die arterielle und venöse Hämoglobinsättigung. Es kommt demzufolge zur Azidose.

Durch Tierexperimente ist nachgewiesen, dass diese Veränderungen der ventilatorischen Grössen durch Wiederherstellung der Integrität des Brustkorbes nachweisbar verbessert werden können. Durch klinische Verläufe wird dies bestätigt.

Es ist bekannt, folgende chirurgische-mechanische Massnahmen neben der Respirator-Behandlung mit positiv endoexpiratorischem Druck in der Behandlung von Thoraxwandbrüchen mit paradoxer Atmung anzuwenden:

Extension,
Osteosyntheseverfahren,
äussere Schienung,
Rippenadaption mit Periostalnähten.

Diesen Behandlungsverfahren haftet entweder der Nachteil der Instabilität oder der Schwierigkeit der Applikation oder beide Nachteile zugleich an.

Der Erfindung liegt daher die Aufgabe zugrunde, ein Implantat zur Behandlung von Thoraxwandbrüchen zu schaffen, mit dem eine gute Stabilität erreicht wird und das zugleich ohne grossen Aufwand und ohne grosse Mühe appliziert werden kann.

Diese Aufgabe wird erfindungsgemäss gelöst durch eine in Querrichtung bogenförmig gekrümmte, längliche, in Längsrichtung leicht verformbare Platte aus körperverträglichem Material, welche an der konkaven Seite scharfe Vorsprünge und an den Längskanten Ausnehmungen aufweist.

Die Vorsprünge, von denen vorzugsweise möglichst viele rippenseitig angeordnet sind, dringen in die Rippen ein. Die Verankerung der Platte erfolgt mit Hilfe von subperiostal durchgeführten Drahtwindungen, deren Enden verzwirbelt werden. Die Ausnehmungen oder Einkerbungen an den Längskanten der Platte dienen zur Aufnahme der Drahtwicklungen.

Die erfindungsgemässe Rippenplatte besitzt eine ausreichende Dicke, um die gewünschte Stabilität bereitzustellen. Anderseits muss das Material, das beispielsweise rostfreier Stahl ist, in Längsrichtung verformbar sein, damit die Platte durch Biegung in Längsrichtung der Rippenform angepasst werden kann.

Zur Anpassung an unterschiedliche Vorfälle ist es vorteilhaft, verschiedene Plattengrössen vorzusehen. In vorteilhafter Weise werden drei verschiedene Grössen vorgesehen, wobei die kleine Grösse eine Plattenlänge von 40 mm, die mittlere Platte eine Länge von 60 mm und die grosse Platte eine Länge von 80 mm aufweist bei einer Plattenbreite von 14 mm für alle Grössen.

Die rippenseitigen Vorsprünge sind so auszubilden, dass sie in das Knochenmaterial eingedrückt werden können. Anderseits müssen sie jedoch ausreichend stabil sein, um eine ausreichende Verankerung zu gewährleisten. Eine Ausgestaltung der Neuerung sieht hierzu vor, dass die scharfen Vorsprünge von schlanken, kegelförmigen Dornen gebildet sind. Im unteren, der Platte zugekehrten Bereich sind die Dorne zweckmässigerweise zylindrisch geformt.

Zur Anordnung der Dorne an der Platte sieht eine weitere Ausgestaltung der Erfindung vor, dass eine Reihe von Dornen in der Längsmitte der Platte angeordnet ist und an den Enden der Platte jeweils zwei beabstandete Dorne vorgesehen sind, die auf einer Linie parallel zur Querachse der Platte liegen und deren Achsen zur Längsmitte der Platte konvergieren.

Weiter oben wurde bereits erwähnt, dass die Befestigung der Rippenplatte mit Hilfe von Draht erfolgt, der um die Rippe und die Rippenplatte herumgeschlungen wird. Zur Festigung der Rippenplatte weist diese Ausnehmungen auf, die vorzugsweise gemäss weiteren Ausgestaltungen der Erfindung an beiden Längskanten paarweise einander gegenüberliegen und deren Kanten bogenförmig gekrümmt sind. Die Ecken der Platte können abgerundet sein.

Zwei Ausführungsbeispiele der Erfindung sollen nachfolgend anhand von Zeichnungen näher beschrieben werden.

Fig. 1 zeigt annähernd massstabgerecht eine Rippenplatte nach der Erfindung in Draufsicht auf die konkave Seite.

Fig. 2 zeigt die Seitenansicht der Rippenplatte nach Fig. 2 in doppelter Grösse.

Fig. 3 zeigt die Endansicht der Rippenplatte nach Fig. 2.

Fig. 4 zeigt eine Rippenplatte mittlerer Grösse nach der Erfindung in Draufsicht auf die konkave Seite.

Fig. 5 zeigt die Seitenansicht der Platte nach Fig. 4 in doppelter Grösse.

Fig. 6 zeigt die Endansicht der Rippenplatte nach Fig. 5.

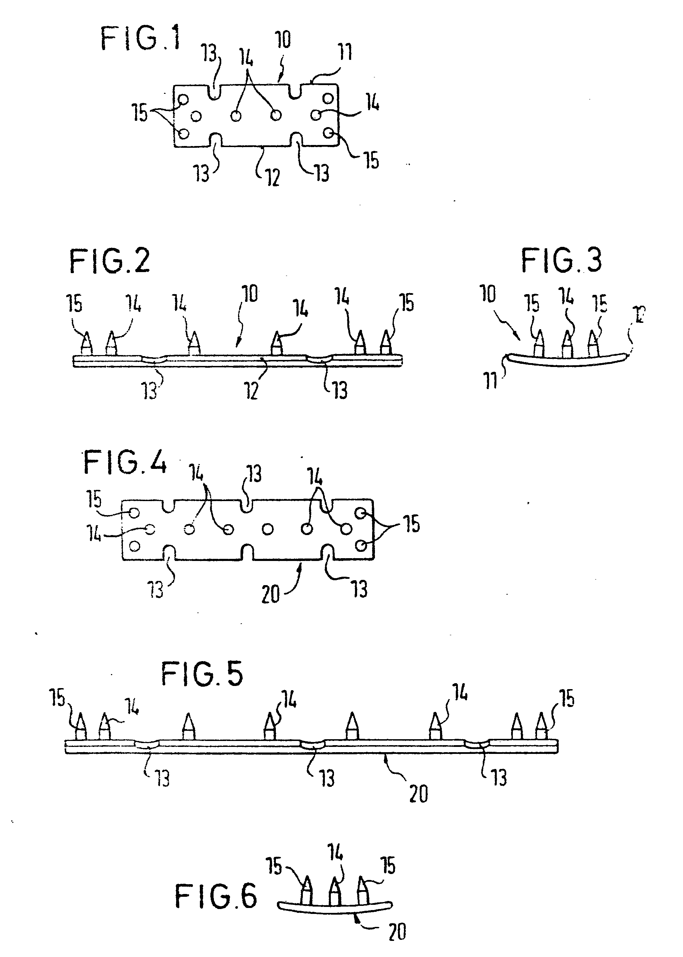

Zunächst sei auf die Fig. 1 bis 3 Bezug genommen. Sie zeigen eine Rippenplatte 10 aus rostfreiem Stahl mit einer Dicke von etwa 1 mm. Die Länge der Platte 10, welche in Querrichtung kreisbogenförmig gekrümmt ist mit einem Radius von etwa 30 mm, beträgt 40 mm, während die Breite 14 mm beträgt. Die rechteckige Platte 10 ist an den Ecken abgerundet. Alle übrigen Kanten sind ebenfalls abgerundet, wobei zu den Längskanten 11, 12 eine geringe Materialverjüngung vorliegt. In den Längskanten 11, 12 sind ausserdem jeweils zwei im Abstand voneinander liegende Aussparungen 13 vorgesehen, die einander paarweise gegenüberliegen und deren Kanten ebenfalls kreisbogenförmig gekrümmt sind.

Entlang der Längsmittellinie befinden sich auf der konkaven Seite der Platte 10 (siehe Fig. 3) vier im gleichmässigen Abstand voneinander angeordnete Dorne 14, deren unterer Abschnitt zylindrisch und deren oberer Abschnitt kegelförmig geformt ist. Die Höhe der Dorne beträgt annähernd 3–4 mm. An den Enden der Platte 10 sind ausserdem je zwei Dorne 15 angeordnet, die auf einer Linie parallel zur Querachse liegen. Wie aus Fig. 3 zu entnehmen, ist die Form der Dorne 15 gleich der der Dorne 14, sie sind jedoch etwas geneigt angeordnet derart, dass ihre Achsen in Richtung zur Längsmitte der Platte konvergieren.

Von der Applikation muss die Platte 10 auch in Längsrichtung gebogen werden, um sie der Rippe anzupassen. Das für die Platte 10 verwendete Material lässt eine gewünschte bleibende Verformung ohne weiteres zu.

Die Einkerbungen oder Ausnehmungen 13 dienen zur Aufnahme einer oder mehrerer Drahtwindungen, mit welchen die Platte 10 verankert wird. Die Drahtenden werden miteinander verzwirbelt. Es sei jedoch bemerkt, dass auch eine andere Befestigungsmöglichkeit denkbar ist.

Die in den Fig. 4 bis 6 dargestellte Rippenplatte 20 gleicht in der Konstruktion der Platte 10 nach den Fig. 1 bis 3, so dass gleiche konstruktive Merkmale mit gleichen Bezugszeichen versehen sind. Die Unterschiede liegen lediglich in den Abmessungen. Die Platte 20 ist 60 mm lang, jedoch auch nur 14 mm breit. Ausserdem sind entsprechend mehr Dorne 14 vorgesehen, nämlich 6 Dorne 14. Auch die Anzahl der einander paarweise gegenüberliegenden Ausnehmungen 13 ist um eine erhöht und beträgt nun 3. Der Einsatz der Rippenplatte 20 entspricht jedoch wiederum der im Zusammenhang mit der Rippenplatte 10 beschriebenen Methode.

**Patentansprüche**

1. Implantat zur Fixation von Rippenbrüchen, gekennzeichnet durch eine in Querrichtung bogenförmig gekrümmte, längliche, in Längsrichtung leicht verformbare Platte (10, 20) aus körperverträglichem Material, welche an der konkav gekrümmten Seite scharfe Vorsprünge (14, 15) und an den Längskanten (11, 12) Ausnehmungen (13) aufweist.

2. Implantat nach Anspruch 1, dadurch gekennzeichnet, dass die scharfen Vorsprünge von schlanken, kegelförmigen Dornen (14, 15) gebildet sind.

3. Implantat nach Anspruch 2, dadurch gekennzeichnet, dass eine Reihe von Dornen (14) in der Längsmitte der Platte (10, 20) angeordnet ist und an den Enden der Platte (10, 20) jeweils zwei beabstandete Dorne (15) vorgesehen sind, die auf einer Linie parallel zur Querachse der Platte (10, 20) liegen und deren Achse zur Längsmitte der Platte (10, 20) konvergieren.

4. Implantat nach einem der Ansprüche 1 bis 3, dadurch gekennzeichnet, dass die Kanten der Ausnehmungen (13) bogenförmig gekrümmt sind.

5. Implantat nach einem der Ansprüche 1 bis 4, dadurch gekennzeichnet, dass die Ausnehmungen (13) an beiden Längskanten (11, 12) paarweise einander gegenüberliegen.

6. Implantat nach einem der Ansprüche 1 bis 5, dadurch gekennzeichnet, dass die Ecken der Platte (10, 20) abgerundet sind.

**Claims**

1. An implant for the fixation of costal fractures, characterized by an elongated plate (10, 20) curved in the transverse direction in the form of an arc and easily shapable in the longitudinal direction, said plate consisting of a material which is tolerated by the body, and being provided with acute projections (14, 15) on the concavely curved side, and with recesses (13) on the longitudinal edges (11, 12).

2. An implant according to claim 1, characterized in that the acute projections are formed by slim, conical thorns (14, 15).

3. An implant according to claim 2, characterized in that there are arranged a series of thorns (14) in the longitudinal center of the plate (10, 20) and in that there are respectively provided at the ends of the plate (10, 20) two thorns (15) spaced from each other which are disposed on a line extending in parallel with the traverse axis of the plate (10, 20) and the axes of which are converging towards the longitudinal center of the plate (10, 20).

4. An implant according to any one of the claims 1 to 3, characterized in that the edges of the recesses (13) are curved in the form of an arc.

5. An implant according to any one of the claims 1 to 4, characterized in that the recesses (13) are disposed in pairs opposite each other on the two longitudinal edges (11, 12).

6. An implant according to any one of the claims 1 to 5, characterized in that the corners of the plate (10, 20) are rounded off.

**Revendications**

1. Implant pour fixation de fracture costale, caractérisé en ce qu'il comprend une plaque (10, 20) incurvée en forme d'arc dans le sens transversal, de forme allongée et facilement déformable dans le sens longitudinal, en matériau toléré par l'organisme, qui comporte des saillies aiguës (14, 15) sur le côté incurvé concave et des échancrures (13) sur les bords longitudinaux (11, 12).

2. Implant selon la revendication 1, caractérisé en ce que les saillies aiguës sont constituées par des pointes ou épines (14, 15) minces et de forme conique.

3. Implant selon la revendication 2, caractérisé en ce qu'une série de pointes (14) sont disposées dans le plan médian longitudinal de la plaque (10, 20) et en ce qu'on a prévu à chacune des deux extrémités de la plaque (10, 20) deux pointes (15) écartées l'une de l'autre, qui sont situées sur une ligne parallèle à l'axe transversal de la plaque (10, 20) et dont les axes convergent vers le plan médian longitudinal de la plaque (10, 20).

4. Implant selon l'une des revendications 1 à 3, caractérisé en ce que les bords des échancrures (13) sont incurvés en forme d'arc.

5. Implant selon l'une des revendications 1 à 4, caractérisé en ce que les échancrures (13) se trouvent en regard l'une de l'autre par paires sur deux bords longitudinaux (11, 12).

6. Implant selon l'une des revendications 1 à 5, caractérisé en ce que les coins de la plaque (10, 20) sont arrondis.

FIG.1

FIG.2

FIG.3

FIG.4

FIG.5

FIG.6